# EUROPÄISCHE PATENTANMELDUNG

(11) **EP 0 829 722 A2**
(43) Veröffentlichungstag der Anmeldung: **18.03.1998**
(21) Anmeldenummer: 97113255.0
(22) Anmeldetag: 31.07.1997
(51) Int. Cl.: G01N 33/543, G01B 7/34

(54) **Verfahren zum Wirkstoff-Screening unter Einsatz der Rasterkraft-Mikroskopie**

(30) Priorität: 02.08.1996 DE 19631326
(71) Anmelder: BASF AKTIENGESELLSCHAFT, 67056 Ludwigshafen (DE)
(72) Erfinder: Friedrich, Thomas, Dr., 64283 Darmstadt (DE); Schrepp, Wolfgang, Dr., 69118 Heidelberg (DE); Akari, Sabri, Dr., 14195 Berlin (DE)
(74) Vertreter: Isenbruck, Günter, Dr.

(57) **Zusammenfassung**

Verfahren zum Wirkstoff-Screening, wobei man den Wirkstoff über die mittels einer Rasterkraft-Mikroskopiervorrichtung (SFM-Vorrichtung) beobachtbare Änderung eines Signals [S] nachweist, sowie Einrichtung, umfassend eine Rasterkraft-Mikroskopiervorrichtung, eine Einzel- oder Mehrfach-Probenaufnahmevorrichtung und eine Steuereinrichtung zum Ansteuern der einzelnen Kompartimente der Einzel- oder Mehrfach-Probenaufnahmevorrichtung.

## Beschreibung

Die Erfindung betrifft ein Verfahren zum Wirkstoff-Screening, insbesondere ein Verfahren zum Massen-Screening im Wirkstoffbereich. Des weiteren betrifft die Erfindung die Verwendung einer Rasterkraft-Mikroskopiervorrichtung (SFM-Vorrichtung) sowie eine Einrichtung, umfassend diese Rasterkraft-Mikroskopiervorrichtung und eine Steuervorrichtung. Außerdem betrifft die Erfindung eine Mehrfach-Probenaufnahmevorrichtung zur Verwendung bei dem vorstehend genannten Verfahren.

Das Finden neuer Leitstrukturen, d.h. von molekularen Strukturen wie etwa Liganden, Agonisten oder Wirkstoffen, die bezüglich ihrer Wirkung auf Enzyme oder Rezeptoren o.ä. Makromoleküle optimiert sind, ist auch heute noch in vielen Teilen ein Zufallsprozeß.

Denn dabei müssen sehr viele - etwa 100.000 bis 1.000.000 - Substanzen durchgemustert werden, bis aktive Substanzen gegen das Zielprotein gefunden werden. Viele Substanzen auf ihre Bioaktivität hin zu untersuchen, dauert sehr lange, wenn man an Aktivitätstests mit z.B. Zellen oder Enzymen denkt. Eine derartig große Substanzzahl kann daher nur dann schnell geprüft und untersucht werden, wenn der Test hochempfindlich und auch sehr schnell ist. Eine Beschleunigung kann beispielsweise dadurch erreicht werden, daß bei dem Test - wie bei den modernen ELISA-Verfahren üblich - im 96-Loch-Mikrotiterformat gearbeitet wird.

In jüngster Zeit kam es mit der Entdeckung der Rastertunnel-Mikroskopie (STM) [G. Binnig, H. Rohrer, C. Gerber, E. Weibel; Phys. Rev. Lett. 49 (1982) 57] zu einer stürmischen Entwicklung auf dem Gebiet der höchst sensitiven sog. Rastersondenverfahren. Zu diesen Rastersondenverfahren gehört auch die Rasterkraft-Mikroskopie (SFM) [G. Binnig, C.F. Quate, C. Gerber; Phys. Rev. Lett. 56 (1986) 930] und die rasteroptischen Nachweisverfahren (SNOM) [E. Betzig, J.K. Trautman; Science 257 (1992) 189]. Bei der Rasterkraft-Mikroskopie wird eine an einem Hebelarm befindliche Spitze zeilenförmig über die zu untersuchende Probe geführt und die Auslenkung des Hebelarms in Abhängigkeit von der Probentopographie über eine Art Lichtzeiger gemessen.

Je nach Fragestellung und Probenbeschaffenheit gibt es verschiedene Betriebsarten, die es erlauben, spezielle Eigenschaften des zu untersuchenden Materials nachzuweisen [S.N. Margonov, M.-H. Whangbo: Surface Analysis with STM and AFM, VCH Verlagsgesellschaft mbH, Weinheim 1996]. So lassen sich Elastizitäts- und Reibungsunterschiede, aber auch strukturell chemische Unterschiede nachweisen [S. Akari, D. Horn, H. Keller, W. Schrepp; Adv. Mater. 7 (1995) 549]. Da die Rasterkraft-Mikroskopie unter Umgebungsbedingungen und auch unter Wasser einsetzbar ist, gibt es einige Untersuchungen, die biologische Fragestellungen betreffen [C. Bustamante, D. Keller; Physics Today, December 1995, 23]. Bei spezieller Präparation der Meßsonde lassen sich auch die Wechselwirkungen zwischen biologischen Modellsystemen direkt nachweisen [E.-L. Florin, V. Moy, H. Gaub; Science 264 (1994) 415]. Bei den dort beschriebenen Untersuchungen wurde das System Biotin/Avidin ausgewählt. Das Biotin wurde an das Serumprotein BSA (Rinder-Serumalbumin) gebunden. Das letztere wiederum wurde unspezifisch an die Silicumnitritspitze eines Rasterkraft-Miltroskops gebunden. Als Probe wurden biotinylierte Agarosekügelchen, die mit Avidin belegt waren, verwendet. Avidin weist vier Bindungsstellen für Biotin auf. Auf diese Weise gelang es, Bindungskräfte zwischen den beiden Spezies zu messen, d.h. Ligand/Rezeptor-Wechselwirkungen sind direkt nachweisbar. Ein Einsatz im Bereich von Immunoassays wurde in WO 92/1570 angeregt.

Unter Berücksichtigung des vorstehend Gesagten ist es Aufgabe der Erfindung, ein Verfahren zum Wirkstoff-Screening bereitzustellen, bei dem eine Vielzahl von Proben in möglichst kurzer Zeit untersucht werden kann. Dieses Verfahren soll nicht nur zeitsparend sein, sondern auch möglichst wenig Aufwand erfordern.

Diese Aufgabe wird durch ein Verfahren zum Wirkstoff-Screening gelöst, bei dem man den Wirkstoff über die mittels einer Rasterkraft-Mikroskopiervorrichtung (SFM -Vorrichtung), die vorzugsweise einen piezoelektrischen Hebelarm aufweist, beobachtbare Änderung eines Signals [S] nachweist.

Gegenstand der Erfindung ist auch die Verwendung einer Rasterkraft-Mikroskopiervorrichtung in diesem Screening-Verfahren sowie eine Einrichtung, umfassend eine Rasterkraft-Mikroskopiervorrichtung, eine Einzel- oder Mehrfach-Probenaufnahmevorrichtung und eine Steuereinrichtung zum Ansteuern der einzelnen Kompartimente der Einzel- oder Mehrfach-Probenaufnahmevorrichtung. Außerdem ist auch Gegenstand der Erfindung eine Mehrfach-Probenaufnahmevorrichtung, umfassend ein Substrat sowie mehrere Probenaufnahmestellen.

Bevorzugte Ausgestaltungen der Erfindung sind Gegenstand der Unteransprüche.

Es zeigen die Figuren:
- Fig. 1: zeigt eine schematische Darstellung des für die Messung der Wechselwirkung verwendeten Systems:
- Fig. 1a): Gezeigt ist eine mit einem Wirkstoff 3 modifizierte Spitze 1, die sich über den immobilisierten Rezeptoren 5 befindet.
- Fig. 1b): Bei Annäherung der modifizierten Spitze 1 an das Substrat 4 findet eine Wechselwirkung statt, die sich mittels einer sog. Kraft-/Abstandskurve (F/d) nachweisen läßt.
- Fig. 1c): Bei Absättigung der Bindungsstelle des Rezeptors 5 mit einem Wirkstoff 3', der mit der Bindungsstelle des Rezeptors 5 in Wechselwirkung tritt und mit dem Wirkstoff 3 identisch oder davon verschieden ist, vor Annäherung der Spitze 1 findet keine spezifische Wechselwirkung statt. Als Nebeneffekt sind lediglich unspezifische Wechselwirkungen denkbar.
- Fig. 2: zeigt Kraft-/Abstandskurven (Kraft F [willkürliche Einheiten, a.u.] vs. Abstand d [nm]) für das System Biotin/Avidin; dargestellt ist jeweils der Teil der Kurve, wie er sich beim Entfernen der Spitze vom Substrat ergibt. Im oberen Teil (m) ist der Fall dargestellt, daß die Avidinoberfläche mit Biotin gesättigt ist. Man findet eine geringe Wechselwirkung. Im unteren Teil (n) ist eine deutlich stärkere spezifische Wechselwirkung zur unbelegten Avidinoberfläche nachweisbar.
- Fig. 3: zeigt als weiteres Beispiel Kraft-/Abstandskurven (F/d) des Thrombinsystems. Der obere Teil (o) zeigt die Kraft-Abstandskurve bei Vorliegen eines gesättigten Systems, der untere Teil (p) diejenige Kurve für die spezifische Wechselwirkung zwischen dem Thrombininhibitor und Thrombin.

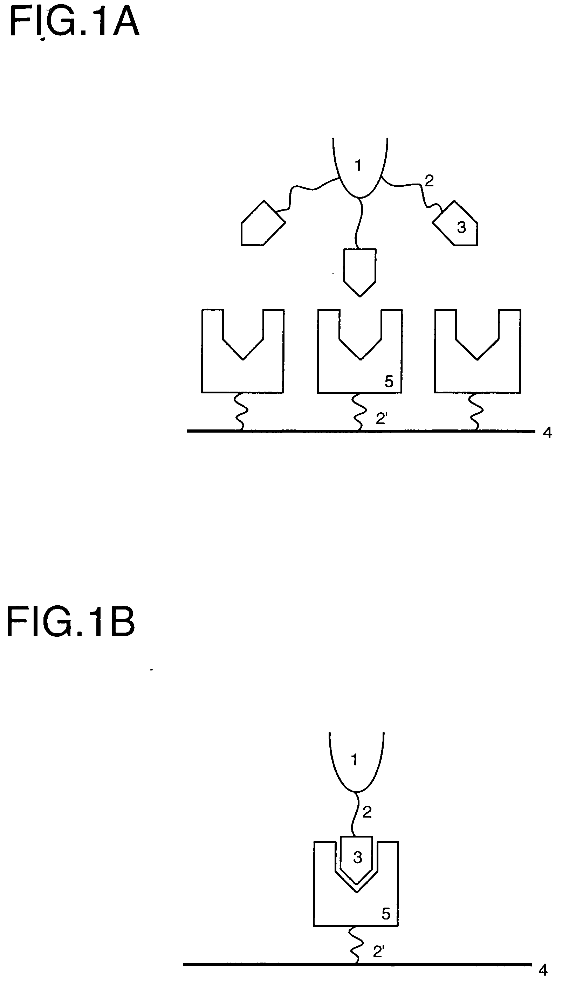

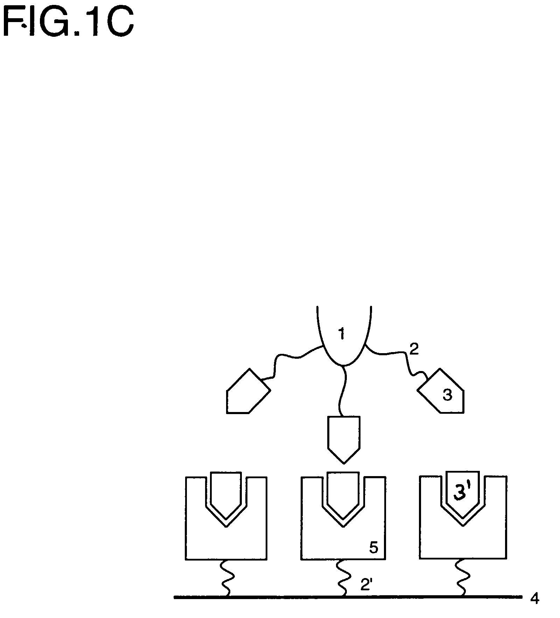

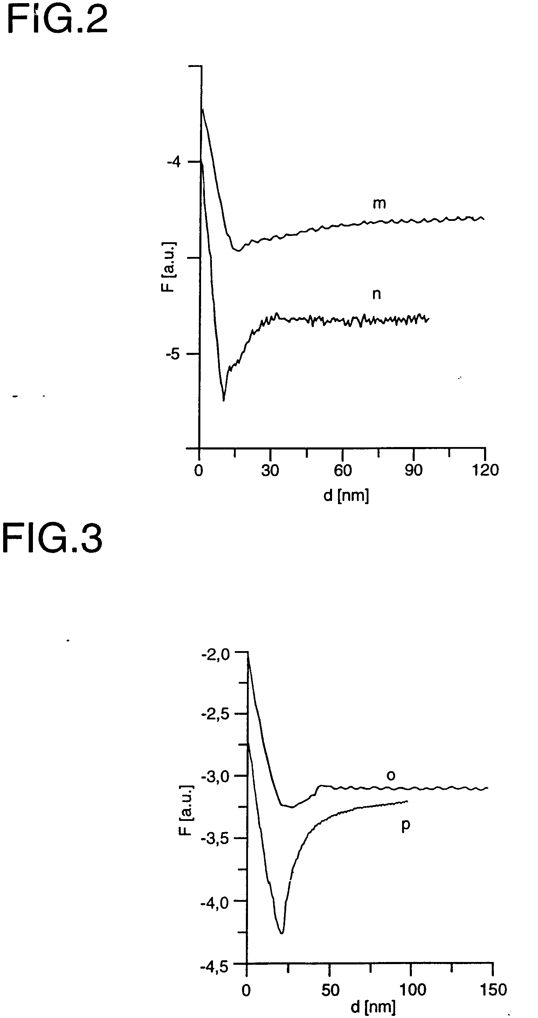

Wie bereits erwähnt, ist auch Gegenstand der Erfindung ein Verfahren zum Wirkstoff-Screening, insbesondere im Bereich des Massen-Screenings, bei dem die Rasterkraft-Mikroskopie bzw. eine entsprechende Rasterkraft-Mikroskopiervorrichtung zum Einsatz kommen. Durch Anbindung des Referenzwirkstoffs, der zu untersuchenden Substanz oder dem Liganden o.ä. an die Meßspitze eines Rasterkraft-Mikroskops wird eine Sonde für das Wirkstoff-Screening im Pharma- und Planzenschutzbereich bereitgestellt. Mit der Sonde wird die zu untersuchende Wechselwirkung zwischen Sonde und -beispielsweise- einem Rezeptor anhand sog. Kraft-/Abstandskurven nachgewiesen. Dabei (vgl. Fig. 1) wird die modifizierte Spitze dem zu untersuchenden Substrat mit dem darauf befindlichen Rezeptor angenähert; als Maß für die Kraft dient dabei die Auslenkung des Hebelarms. Diese wird grafisch (vgl. Fig. 2 und 3) als Kraft F [willkürl. Einheiten, a.u.] in Abhängigkeit von dem Abstand d [nm] zur Probe aufgetragen. Bei Verwendung geeichter Spitzen kann die Kraft auch absolut angegeben werden. Grundsätzlich zeigen die derartig ermittelten Kraft-/Abstandskurven einen absteigenden und einen aufsteigenden Ast (Fig. 2). Dieser Verlauf wird nachfolgend für einen Meßzyklus beschrieben: Bei sehr geringen Abständen von der Probe erfährt die Spitze eine von der chemischen und elektronischen Zusammensetzung der Probe abhängige anziehende Kraft (rechter Teil der Kurven in Fig.2 und 3). Bei weiterer Annäherung der Spitze an die Probenoberfläche erfährt die Spitze eine Abstoßung (repulsive Kraft); dies wird im linken Teil der Kurven in Fig. 2 und 3 dargestellt. Beim Zurückziehen der Spitze verringert sich dementsprechend die repulsive Kraft. Beim weiteren Zurückziehen bleibt die Spitze aufgrund anziehender Wechselwirkungskräfte quasi am Substrat haften. Bei noch größeren Abständen nimmt der Hebelarm dann wieder einen stabilen Zustand ein. Als Maß für die attraktive Wechselwirkung wird die Fläche der Kurve unter der Kraft-Null-Linie genommen, genauer gesagt die Schnittfläche der ermittelten Kurve mit der parallel zur Abszisse und durch den Null-Wert der Ordinate verlaufenden Linie.

Die größten Unterschiede in den Kraft-/Abstandskurven werden beim Wegziehen des Hebelarms von der Probenoberfläche registriert. Man kann jedoch auch davon ausgehen, daß bei speziellem Hebelarmdesign, z.B sehr dünnen Hebelarmen oder Hebelarmen auf piezoelektrischer Basis mit z.B. sehr geringer Kraftkonstante nur der Ausschlag des Hebelarms infolge der attraktiven Wechselwirkung detektiert wird.

Für das Wirkstoff-Screening läßt sich die Rasterkraft-Mikroskopie beispielsweise vorteilhaft in der folgenden Weise einsetzen:

Zunächst wird ein Rezeptor 5 (Fig. 1) auf einer geeigneten Oberfläche eines Substrats 4 immobilisiert. Dieser zu immobilisierende Stoff wird nachfolgend auch als Molekül A bezeichnet. Bei dem Substrat kann es sich um einen Siliciumwafer handeln, der vorzugsweise mit Gold bedampft ist. Aber auch andere Substrate wie insbesondere Glimmer, Graphit, Polymere oder durch Vorbehandlung, wie Plasma-, Coronabehandlung oder Adsorption modifizierte Substrate sind geeignet.

Die Immobilisierung des Rezeptors bzw. des Moleküls A auf dem Substrat kann unmittelbar mittels Adsorption erfolgen. Bevorzugt ist es jedoch, das zu untersuchende Molekül mittels eines Abstandhalters 2' auf der Substratoberfläche zu immobilisieren. Bevorzugte Abstandhalter sind Aminohexansäure oder Spermidin oder ein weiterer Ligand des zu untersuchenden Moleküls A. Diese Abstandhalter ("spacer") können direkt an die Substratoberfläche adsorbiert werden. Bevorzugt ist es jedoch, auf der Substratoberfläche, beispielsweise der Oberfläche eines mit Gold bedampften Siliciumwafers zunächst eine Mercaptocarbonsäure, beispielsweise 10-Mercaptodecansäure binden zu lassen. Das Molekül bindet mit der Thiolgruppe an die Goldoberfläche unter Ausbildung einer quasi monomolekularen Schicht. Die so modifizierte Substratoberfläche wird anschließend mit dem vorstehend genannten Abstandhalter, bevorzugt Aminohexansäure oder Spermidin, derivatisiert. Dazu werden die Carboxylgruppen der Mercaptocarbonsäuren zunächst mit bekannten Techniken aktiviert und anschließend mit dem Abstandhalter umgesetzt bzw. derivatisiert.

Zur Aktivierung der Carbonsäuregruppen vor der Umsetzung können übliche Verfahren, wie sie beispielsweise aus der Peptidsynthese bekannt sind, angewendet werden. Beispielsweise ist es möglich, die Carboxylgruppe der Mercaptocarbonsäure mit N-Hydroxysuccinimid (C₄H₅NO₃) oder N-(3-Dimethylaminopropyl)-N'-ethylcarbodiimid(hydrochlorid)(C₈H₁₈ClN₃) 5 Minuten lang in Gegenwart eines Abstandhalters wie Aminohexansäure oder Spermidin umzusetzen. Falls erforderlich, kann dieser Vorgang auch mehrfach wiederholt werden, um die Länge des Abstandhalters zu vergrößern. Wurde Spermidin als Spacer gewählt, so kann auch die noch freie Aminogruppe des Spermidins mit einer aktivierten Carboxylgruppe eines Liganden umgesetzt werden. Die Carboxylgruppe des Liganden wiederum kann, wie vorstehend beschrieben, mit Hilfe von N-Hydroxysuccinimid aktiviert werden. Auf diese Weise kann der Abstand zwischen der Substratoberfläche 4 und dem an den Abstandhalter 2' zu bindenden Molekül A bzw. Rezeptor 5 auf das erwünschte Maß eingestellt werden.

In gleicher Weise wie der Rezeptor 5 an einer Substratoberfläche 4 immobilisiert werden kann, kann auch an der Spitze 1 des Rasterkraft-Mikroskops ein Wirkstoff 3 vorzugsweise mittels eines Abstandhalters 2 immobilisiert werden. Ebenso ist die umgekehrte Anordnung denkbar, d.h der Wirkstoff wird an der Substratoberfläche und der Rezeptor an der Mikroskopspitze immobilisiert

Dazu wird zunächst die Spitze 1 mit einer z.B. ca. 5 nm dicken Schicht Chrom als Haftvermittler und anschließend mit Gold in einer Dicke von ca. 50 nm bedampft. Die mit Gold bedampfte Spitze wird - in gleicher Weise wie in analoger Weise vorstehend für das Substrat beschrieben - zunächst mit einer Mercaptocarbonsäure beschichtet, an der der Abstandhalter 2 angehängt wird. Statt des Chroms als Haftvermittler können auch andere Metalle als Haftungsvermittler verwendet werden. Anstelle von Gold kann auch auf die haftungsvermittelnde Schicht eine andere Schicht aufgebracht sein. Wesentlich ist lediglich, daß es sich dabei um eine Schicht aus einem Material handelt, das im wesentlichen hydrophil ist, da sonst Untersuchungen in wäßrigen Lösungen erschwert werden.

Aufgrund des Meßprinzips ist es besonders vorteilhaft, daß eine Parallelisierung des Verfahrens möglich ist. Es können statt einer Rasterkraftsonde gleichzeitig mehrere Sonden verwendet werden. Dadurch wird eine parallele Auswertung von Mehrfach-Probenaufnahmevorrichtungen, wie z.B. ELISA-Platten möglich. Im Falle der parallelen Auswertung ist es besonders bevorzugt, daß die Rasterkraft-Mikroskopiervorrichtung Hebelarme piezoelektrischer Art verwendet. Dadurch kann eine deutliche Vereinfachung des Aufbaus erreicht werden, da der optische Nachweis der Auslenkung des Hebelarmes entfällt.

Bei der Mehrfachprobenaufnahmevorrichtung handelt es sich bevorzugt um eine solche, deren Abmessungen zwecks Erhöhung der Geschwindigkeit des erfindungsgemäßen Wirkstoff-Screening-Verfahrens miniaturisiert sind. In diesem Falle ist die Untergrenze für die Größe (Durchmesser) der einzelnen Kompartimente lediglich durch die Größe der Spitze festgelegt. Die Untergrenze liegt dadurch bei etwa 10 nm.

Nach oben hin gibt es grundsätzlich keine Begrenzung. In Hinsicht auf die Optimierung der Geschwindigkeit des Screening-Verfahrens ist es aber nicht sehr effektiv, wenn die Größe des Kompartiments bei der miniaturisierten Vorrichtung einige 100 µm, insbesondere 500 µm überschreitet. Eine bevorzugte obere Grenze liegt bei 150 µm.

Bei einer derartig miniaturisierten Aufnahmevorrichtung ist es für die Optimierung der Geschwindigkeit auch nicht mehr nötig, daß das Einzelkompartiment, das die Probe enthält, die Form eines Lochs ["well"] aufweist, wie sie von den üblichen Mikrotiterplatten bekannt ist. Als Strukturen können z.B. durch Lithographie und/oder Ätz- sowie Stempelprozesse hergestellte Vertiefungen, Näpfchen oder auch metallische Erhebungen z.B. in oder auf Silicium oder anderen Materialien wie z.B Kunststoffen oder Glas dienen. Denkbar ist auch die Verwendung von Aufdampfstrukturen oder solchen, die durch Self-Assembly-Verfahren hergestellt werden. Auch nach dem LIGA-Verfahren hergestellte Mikrostrukturen können verwendet werden. Derartig ausgebildete Probenaufnahmestellen weisen eine Größe bis in den nm-Bereich hinunter auf.

Zum noch schnelleren Ablesen eines Kompartiments reicht es aus, wenn dieses die Form eines Napfs, Trogs oder einer flachen Vertiefung aufweist. Die in dieser Hinsicht vorteilhafteste Ausgestaltung der bei dem erfindungsgemäßen Verfahren bzw. der erfindungsgemäßen Einrichtung eingesetzten Mehrfachprobenaufnahmevorrichtung besteht einfach aus einer sehr flachen planaren Scheibe, auf der die zu untersuchenden Proben in Form von kleinen Tröpfchen aufgesetzt sind. Falls als Substrat ein Si-Wafer verwendet wird, der mit Gold bedampfte Bereiche aufweist, ist es bevorzugt, daß die einzelnen Kompartimente mit Gold unterlegt sind, während das hydrophobe Silicium die einzelnen Kompartimente voneinander abtrennt.

Generell können die einzelnen Kompartimente einer derartigen Mehrfachprobenaufnahmevorrichtung beliebig angeordnet sein, insbesondere aber in Rechteck- oder Kreisstruktur.

Auf diese Weise kann die Geschwindigkeit des Verfahrens nochmal gesteigert werden. Denn in diesem Falle ist es möglich, die Rasterkraft-Mikroskopiervorrichtung im stationären Zustand zu lassen. Das Auslesen der Proben erfolgt dann in der Weise, daß die Meßsonden der Rasterkraft-Mikroskopiervorrichtung mit der Mehrfach-Probenaufnahmevorrichtung angesteuert werden, ähnlich wie bei den üblichen Lichtmikroskopen nicht die Objektive selbst bewegt werden, sondern der Objekttisch mit dem darauf befindlichen Objektträger das Objektiv ansteuert.

Die Erfindung wird durch die nachfolgenden Beispiele näher erläutert:

### Beispiel 1

Das bekannte System Biotin/Avidin, bei dem Avidin den Rezeptor und Biotin den Liganden darstellt, wurde verwendet. Biotin wurde auf der Spitze immobilisiert, Avidin auf einer entsprechend vorbehandelten Siliciumwafer-Oberfläche (vgl. Fig. 1). Die Konzentration der beiden Stoffe bei der Immobilisierung betrug 100 µg/ml. Das so hergestellte System wurde mittels eines Rasterkraft-Mikroskops (Fa. Topometrix, Darmstadt; Explorer-Meßkopf) untersucht. Die Wechselwirkung zwischen Biotin und Avidin wurde anhand der in Fig. 2 dargestellten Kraft-/Abstandskurve gemessen. Zum Nachweis der Spezifität der Wechselwirkung wurde als Kontrolle (vgl. Fig. 2, obere Hälfte, m) das auf dem Substrat immobilisierte Avidin durch Eintauchen in eine biotinhaltige Lösung abgesättigt. Anschließend wurde die Wechselwirkung des derartig abgesättigten Rezeptors mit der Biotin-Rasterkraft-Mikroskopiersonde gemessen. Wie im oberen Teil, m der Fig. 2 gezeigt, kommt es zwischen der Biotin-Rasterkraft-Mikroskopiersonde und dem mit Biotin abgesättigten Avidin zu keiner spezifischen Wechselwirkung. Die spezifische Wechselwirkung zur unbelegten Avidinoberfläche ist deutlich stärker (Fig. 2, untere Meßkurve, n).

### Beispiel 2

Als weiteres System wurde (Fig. 3) das System Thrombin/Thrombininhibitor mittels der Rasterkraft-Mikroskopiervorrichtung untersucht. Dazu wurde zunächst Thrombin in der vorstehend angegebenen Weise auf dem Substrat immobilisiert. Als Rasterkraft-Mikroskopiersonde wurde der auf der Spitze des Rasterkraft-Mikroskops immobilisierte Thrombininhibitor verwendet. Zum Vergleich der Spezifität der Wechselwirkungen zwischen Substrat (Thrombin) und Spitze (Thrombininhibitor) wurde als Kontrolle (Fig. 3, oberer Teil, o) das Thrombin vor der Messung mit Thrombininhibitor abgesättigt. Das abgesättigte Thrombin zeigte keine spezifische Wechselwirkung mit dem auf der Rasterkraft-Mikroskopierspitze immobilisierten Thrombininhibitor. Die spezifische Wechselwirkung des Thrombininhibitors ist aus Fig. 3 [untere Meßkurve, p] ersichtlich.

## Patentansprüche

1. Verfahren zum Wirkstoff-Screening, wobei man den Wirkstoff über die mittels einer Rasterkraft-Mikroskopiervorrichtung (SFM-Vorrichtung), die vorzugsweise einen piezoelektrischen Hebelarm aufweist, beobachtbare Änderung eines Signals [S] nachweist.

2. Verfahren nach Anspruch 1, wobei das Wirkstoff-Screening die Schritte umfaßt, daß
a) ein Rezeptor (5) an ein Substrat (4) direkt oder indirekt, kovalent oder nicht-kovalent gebunden wird,
b) mittels der Rasterkraft-Mikroskopiervorrichtung ein über das Signal [S] nachweisbarer Wirkstoff (3) kovalent oder nicht-kovalent an den Rezeptor (5) binden gelassen wird,
c) eine einen Wirkstoff (3') enthaltende Lösung hinzugegeben wird und die Anwesenheit des Wirkstoffes (3') über die Änderung der mittels der Rasterkraft-Mikroskopiervorrichtung gemessenen Bindung des Wirkstoffs (3) an den Rezeptor (5) und damit des Signals [S] nachgewiesen wird und
d) die Schritte a) bis c) an den einzelnen Kompartimenten einer Mehrfach-Probenaufnahmevorrichtung nacheinander oder parallel zueinander vollzogen werden.

3. Verfahren nach Anspruch 1 oder 2, wobei das Signal [S] in Form einer Kraft-/Abstandskurve (F/d) oder eines Teils einer Kraft-/Abstandskurve aufgezeichnet wird.

4. Verfahren nach einem der vorhergehenden Ansprüche, wobei die Bindung des Rezeptors (5) an das Substrat (4) mittels Adsorption direkt oder indirekt über einen Abstandhalter (2'), insbesondere Spermidin oder Aminohexansäure, der vorzugsweise seinerseits über einen an das Substrat adsorbierte Verbindung, insbesondere eine Mercaptocarbonsäure, gebunden wird, erfolgt.

5. Verfahren nach einem der vorhergehenden Ansprüche, wobei eine parallelisierte Meßeinrichtung zum Nachweis mehrerer Meßstellen zum Wirkstoff-Screening eingesetzt wird.

6. Verfahren nach einem der vorhergehenden Ansprüche, wobei es sich bei dem Substrat (4) um einen - wahlweise mit Au bedampften - Si-Wafer, eine Mehrfach-Probenaufnahmevorrichtung, Glimmer, Graphit oder um eine zelluläre Struktur, insbesondere um membranfixierte Rezeptoren, oder eine Kombination derselben handelt.

7. Verwendung einer Rasterkraft-Mikroskopiervorrichtung (SFM-Vorrichtung) zum Wirkstoff-Screening, wobei man den Wirkstoff über die mittels der Rasterkraft-Mikroskopiervorrichtung beobachtbare Änderung eines Signals [S] nachweist.

8. Einrichtung, umfassend eine Rasterkraft-Mikroskopiervorrichtung, eine Einzel- oder Mehrfach-Probenaufnahmevorrichtung und eine Steuereinrichtung zum Ansteuern der einzelnen Kompartimente der Einzel- oder Mehrfach-Probenaufnahmevorrichtung, wobei die einzelnen Kompartimente der Einzel- oder Mehrfach-Probenaufnahmevorrichtung mit der Rasterkraft-Mikroskopiervorrichtung oder die Rasterkraft-Mikroskopiervorrichtung mit der Einzel- oder Mehrfach-Probenaufnahmevorrichtung bevorzugt parallelisiert angesteuert wird.

9. Mehrfach-Probenaufnahmevorrichtung, umfässend ein Substrat sowie mehrere mikrostrukturierte Probenaufnahmestellen.

10. Mehrfach-Probenaufnahmevorrichtung nach Anspruch 9, wobei die mikrostrukturierten Probenaufnahmestellen die Form flacher Näpfe zeigen.
